# EUROPEAN PATENT SPECIFICATION

(11) **EP 0 500 691 B1**
(45) Date of publication and mention of the grant of the patent: **25.01.1995**
(21) Application number: 90916985.6
(22) Date of filing: 31.10.1990
(51) Int. Cl.: A61K 7/16, A61K 7/18

(54) **STABILIZED STANNOUS FLUORIDE COMPOSITIONS FOR ORAL CARE**
STABILISIERTE ZINNFLUORIDMITTEL ZUR MUNDPFLEGE
COMPOSITION DE SOINS BUCCAUX EN FLUORURE STANNEUX STABILISE

(30) Priority: 15.11.1989 US 437467; 15.11.1989 US 437469; 15.11.1989 US 437470
(43) Date of publication of application: 02.09.1992
(73) Proprietor: THE GILLETTE COMPANY, Boston, Massachusetts 02199 (US)
(72) Inventor: SUHONEN, Christopher, H., San Jose, CA 95125 (US)
(74) Representative: Baillie, Iain Cameron
(86) International application number: US9006340
(87) International publication number: WO9107163

(56) References cited:
- EP-A- 0 321 650
- EP-A- 0 333 301
- US-A- 4 366 146
- US-A- 4 960 586
- US-A- 4 961 924
- US-A- 5 015 467

## Description

This invention relates to aqueous stannous fluoride compositions for oral care with increased stannous ion stability. In particular, this invention is directed to compositions comprising stannous fluoride stabilized with a copolymer of maleic anhydride or acid and a lower alkyl vinyl ether. The compositions of the invention are particularly useful in the form of aqueous gel compositions, aqueous mouthwash compositions and aqueous toothpaste compositions.

Stannous fluoride has been used in oral care products since the early 1950's, and stannous fluoride has been reported to be an effective agent for treating various oral conditions and diseases including plague, gingivitis, sensitivity, enamel decalcification, and periodontitis, among others. Product stability is an outstanding problem because stannous fluoride is unstable in water and forms stannous oxy-fluoride and stannic compounds, both of which reduce or inhibit enamel fluoridation, that is, formation of stannous fluorophosphate and fluorapatite. This instability has required formulation of home treatment gels as anhydrous gels. Despite highly specialized precautionary measures during formulation of oral care products, significant variations are found in stannous ion concentrations of commercial dentifrice gel products among different manufacturers as well as among batches of the same brand of dentifrice because of stannous ion instability.

The original aqueous stannous fluoride formulations have been reformulated as stannous ion-free compositions because of the instability of the stannous ion in water. Numerous stannous fluoride formulations with "stabilizing agents" have been proposed.

U.S. Patent 3,445,567 claims that aqueous stannous fluoride compositions can be stabilized with sorbitol or a mixture of sorbitol and glycerine. However, this composition lacks the shelf-life stability required for commercial use.

U.S. Patents 3,711,604, 3,919,409, 3,935,306 and 3,980,767 disclose toothpaste and dentifrice formulations including soluble fluoride compounds, including stannous fluoride. The toothpaste formulations use a variety of standard ingredients and gelling agents including carboxyvinyl polymers sold under the tradename Carbopol and insoluble abrasives such as silica and silicates. The stannous fluoride containing embodiments disclosed in these patents lack the stability found in the compositions of this invention.

U.S. Patent 4,418,057 describes another approach. Stannous fluoride is formulated as a non-aqueous gel mixture including anhydrous glycerin and hydroxyethyl cellulose gelling agent. Total exclusion of moisture from the moisture is required to protect the stannous ion.

U.S. Patent 4,259,316, while noting the instability of stannous fluoride, proposes a compositions containing phytic acid for inhibiting dental caries. Both dry dentrifices and aqueous gel and mouthwash formulations are described. If protected from moisture, the dentrifices would appear to retain the stannous ion in efficacious form. The stannous ion would rapidly convert to the ineffective oxy-stannous or stannate forms upon exposure to moisture in solution or in the oral cavity. Apparently, excess amounts of stannous fluoride are added in an effort to offset the loss of stannous ion. However, this requires the exposure of the user to excessive, unsafe levels of stannous ion.

EPO application 88308337.0 (Publication No. 0 311 260 A3) published April 12, 1989 describes compositions containing stannous fluoride, and as a stannous reservoir to replace stannous ion lost through degradation, stannous gluconate. Mouthwashes are described. Listed as suitable abrasive polishing agents are silicas, including gels and precipitates, insoluble sodium polymetaphosphate; β-phase calcium pyrophosphate; alumina and resinous abrasive materials which do not contribute soluble calcium ions which form complexes with the fluoride ion. Calcium carbonate, calcium phosphate and regular calcium pyrophosphate are excluded.

U.S. Patent 4,254,101 describes compositions containing a humectant, silica abrasive, a carboxyvinyl polymer, water and fluoride compounds as optional ingredients. The only carboxyvinyl polymers disclosed are colloidally water-soluble polymers of acrylic acid crosslinked with polyallyl sucrose or polyallyl pentaerythritol. A variety of fluoride compounds including sodium fluoride are described as suitable optional ingredients. Stannous fluoride is included in the list of suitable fluoride compounds although no suggestion is made about its instability or that it could be used as an effective source of stannous ion. It is presented as equivalent to sodium fluoride as source of fluoride ion. Phosphorus-containing anticalculus agents are also listed as optional ingredients.

U.S. Patent 4,515,772 describes oral compositions including dentrifices and aqueous compositions including toothpastes and mouthwashes containing certain pyrophosphate salts as anticalculus formulations. In the prior art description, it lists a number of chelating agents proposed as anticalculus agents, including EDT (ethylenediaminetetraacetic acid), nitrilotriacetic acid, polyphosphonates and fluoride, and carbonyl diphosphonates. The patent lists as suitable abrasives silica, calcium pyrophosphate, β-phase pyrophosphate, alumina and other materials. A comprehensive list of fluoride ion sources are listed. Stannous fluoride is included in the list although no suggestion is made about its instability or that it could be used as an effective source of stannous ion. A variety of flavoring agents are disclosed. Included in a list of binders are gums and carboxyvinyl polymers.

U.S. Patent 3,956,479 describes use of quaternary anticalculus compounds in dental creams, tables or powders containing polishing agents such as water-insoluble phosphates, binders, detergents, gelling agents, flavoring agents, and fluoride-containing compounds such as sodium fluoride, stannous fluoride and sodium monofluorophosphate. Mouthwashes are also described.

U.S. Patent 4,627,977 describes oral paste and gel dentifrice compositions containing a calculus-inhibiting amount of a linear molecularly dehydrated polyphosphate salt, and to inhibit hydrolysis of the polyphosphate salt in the saliva, a combination of a fluoride ion-providing source and a synthetic linear polymeric polycarboxylate. Included in the list of suitable polycarboxylates are copolymers of maleic anhydride or acid and ethylenically unsaturated monomers including alkyl vinyl ethers available as Grantrez AN 139, AN 119, and S97. An extensive list of fluoride ion sources are provided, including sodium and stannous fluorides and sodium monofluorophosphate. An extensive list of polishing agents-are provided including water-insoluble phosphates, silicates and silicas, bentonites and inorganic polishing agents. Silicas are listed as preferred.

A. Gaffar et al in Compend.Contin.Educ.Dent, Suppl. No. 8, 242-250 (1987) and Thomas G. Schiff in Compend.Contin.Educ.Dent, Suppl. No. 8, 275-277 (1987) describe evaluations of pyrophosphate toothpaste compositions containing sodium fluoride and a copolymer of methoxyethylene and maleic anhydride known as "GANTREZ" as anticalculus dentifrices. The oral cavity contains enzymes which attack and degrade the pyrophosphates, rapidly removing their anticalculus activity. The enzymes require soluble magnesium ion for activity. The copolymer is provided to complex magnesium ions, making them unavailable to the enzymes and thus inhibiting their activity. Its purpose is to protect the pyrophosphates and maintain their anticalculus activity.

The present invention provides an aqueous stannous fluoride composition for oral care, containing stannous fluoride and a lower alkyl vinyl ether and maleic anhydride or maleic acid copolymer in an amount sufficient to effectively stabilize the stannous fluoride concentration, the composition being substantially free from soluble pyrophosphate and aldehyde containing flavoring agents.

An embodiment of the invention resides in the stannous fluoride being in combination with a stannous ion chelating copolymer of an alkyl vinyl ether and maleic anhydride in an amount to effectively stabilize the stannous ion.

In an embodiment of the invention, the composition is an aqueous non-abrasive home treatment gel composition. In this embodiment, the composition may contain from 10 to 30 weight percent water, from 0.05 to 5 and preferably from 0.5 to 5 weight percent of the chelating copolymer and from 0.05 to 5 and preferably from 0.3 to 0.5 weight percent stannous fluoride. The weight ratio of stannous fluoride to copolymer is preferably between 0.01 to 1.0. The composition is substantially free from all abrasives and aldehyde group containing flavoring agents.

In another embodiment of the invention, the composition is an aqueous mouthwash. In this embodiment, the composition may contain from 80 to 98 weight percent water, from 0.05 to 5 and preferably from 0.5 to 5 weight percent of the chelating copolymer and from 0.05 to 5 and preferably from 0.05 to 0.5 weight percent stannous fluoride. The weight ratio of stannous fluoride to copolymer is preferably between 0.01 to 1.0. The composition is substantially free from soluble phosphates such as pyrophosphate and aldehyde group containing flavoring agents.

In still another embodiment of the invention, the composition is an aqueous toothpaste. In this embodiment, the composition may contain from 10 to 30 weight percent water, from 0.05 to 5 and preferably from 0.5 to 5 weight percent of the chelating copolymer and from 0.05 to 5 and preferably from 0.3 to 0.5 weight percent stannous fluoride and an insoluble abrasive agent. The weight ratio of stannous fluoride to copolymer is preferably between 0.01 to 1.0. The composition is substantially free from silica, soluble phosphates such as soluble pyrophosphates (i.e., tetrasodium pyrophosphate, tetrapotassium phosphates, etc.), and aldehyde group containing compounds.

The method of this invention for formulating a stabilized aqueous composition for oral care comprises the step of dispersing the stannous fluoride in an aqueous solution of an alkyl vinyl ether and maleic anhydride copolymer, the amount of said copolymer being sufficient to stabilize the stannous fluoride concentration during formulation.

This invention is directed to a stannous fluoride formulation which is "effectively stabilized", that is, the stannous fluoride concentration in the product after three months at forty-five degrees centigrade remains at an acceptable therapeutic level. Product stability after three months as forty-five degrees centigrade is comparable to product stability after three years at room temperature.

The essential ingredients of the compositions of this invention are stannous fluoride and the stannous ion chelating copolymer in an effectively stabilizing amount.

The term "effectively stabilized" and "effectively stabilizing amount" is defined to mean that the stannous ion concentration, expressed as stannous fluoride, after three months storage under the conditions described in Example 3 is equivalent to about 70 percent or more of the original concentration of stannous ion at the time of formulation.

The stannous ion stabilizing copolymer is a copolymer of maleic anhydride or acid and a polymerizable ethylenically unsaturated monomer, preferably a lower alkyl vinyl ether such as methoxyethylene, having a molecular weight of from about 30,000 to 1,000,000. The mole ratios of the maleic anhydride or acid to the ethylenically unsaturated monomer is preferably from 1:4 to 4:1. Suitable polymers are available from GAF under the tradename "GANTREZ" and are disclosed in U.S. Patent 4,627,977. These copolymers have the unique ability to form chelates with the stannous ion which are sufficiently strong to provide oxidation protection to the stannous ion while being sufficiently weak to not remove calcium from the tooth structure. EDTA and other strong chelating agents are undesirable because they deplete calcium from the tooth enamel.

The term "lower alkyl" is defined to be a straight or branch-chained hydrocarbon group having from 1 to 6 carbons and preferably from 1 to 4 carbons and including methyl, ethyl, propyl, isopropyl, butyl, isobutyl, t-butyl, pentyl and hexyl groups.

The compositions of this invention may contain other conventional components such as anti-foaming agents, gelling agents, humectants, flavoring agents, anticariogenic agents, soluble fluoride compounds, surfactants, coloring or whitening agents, antibacterlal agents, preservatives, chlorophyll compounds, and additional ammoniated materials which do not interfere with the stability of the stannous ion by reacting therewith or with the stannous ion chelating polymer. Suitable materials are described in U.S. Patents 4,418,057, 4,254,101 and 4,627,977.

The present compositions which are non-abrasive home treatment gels may also contain conventional aqueous gelling agents.

The compositions which are toothpaste compounds may contain one or more abrasive agents (other than silica or silicates) and can contain other conventional ingredients to impart the desired consistency, texture, resistance to hardening, flavor and the like.

Suitable abrasive agents include insoluble phosphates such as calcium pyrophosphate, β-phase pyrophosphate, and alkali metal metaphosphates. We have discovered that the stabilizing function of the stannous ion chelating polymer is not effective in the presence of silica and silicates.

Any conventional humectant can be used. Suitable humectants include sorbitol, glycerin, or other polyhydric alcohols, the natural or synthetic gums conventionally used as hardening control agents and binders.

Suitable gelling agents for use in the non-abrasive gel composition of this invention include from 0.1 to 10 and preferably from 0.5 to 5 weight percent gelling agent. Gelling agents should be silica and silicate free compounds such as Irish moss, gum tragacanth, starch, polyvinlypyrolidone, hydroxyethyl propylcellulose, hydroxybutyl methyl cellulose, hydroxypropyl methyl cellulose, hydroxyethyl cellulose, sodium carboxymethyl cellulose, and the like.

The compositions can also contain flavoring agents which do not have an aldehyde group. We have discovered that aldehyde-containing flavoring agents such as cinnaldehyde, while not reacting directly with the stannous ion, interact with the stannous ion chelating polymer, allowing stannous oxidation to occur.

The non-abrasive gels may have a pH within the range of from 2 to 11 and preferably from 2.5 to 5.0. The mouthwash compositions of the invention may have a pH within the range of from 2.5 to 1 and preferably from 3 to 7. The toothpaste compositions of the invention may have a pH within the range of from 2 to 11 and preferably from 3 to 7.

The formulations of this invention are manufactured using procedures which protect the stannous ion from oxidation. The stannous fluoride is initially dissolved in an aqueous solution containing the stannous chelating copolymer. This solution may be mixed with other components by conventional procedures to form the compositions of the invention. In a preferred procedure, a premix solution is prepared by dispersing from 0.5 to 26 weight percent stannous fluoride in water containing from 5.0 to 26 weight percent stannous ion stabilizing copolymer, the weight ratios of the stannous fluoride to copolymer being from 0.01 to 1.0 in the premix solution. This solution can be then mixed with other components by conventional procedures to form the formulations of this invention. Additional protection from oxidation can be provided during manufacture by carrying out the procedures in an inert atmosphere.

For additional protection against the oxidation, the product should be stored in oxygen impermeable containers. Preferred containers are laminated tube such as "GLAMINATE" tubes (American Can Company). Suitable tubes are laminates of a flexible plastic such as a polyolefin (i.e., low density polyethylene, etc.) and an oxygen barrier layer such as an oxygen impermeable metal foil (i.e., aluminum foil). The laminates include other conventional layers such gas adhesives (i.e., ethylene copolymers), paper or other non-woven fibrous materials, and the like. For toothpaste, the layer exposed to the stannous fluoride toothpaste should be essentially free from toxic substances and materials which would be released into the toothpaste during manufacture, storage or use and impair the stability of the toothpaste composition.

A preferred gel formulation can have the formulation shown in Table A-1.

**TABLE A-1**

| Ingredients | Weight Percent |
|---|---|
| Purified Water USP | 10 - 20 |
| Sorbitol Solution (70%) USP | 50 - 80 |
| Stannous chelating copolymer | 0.5 - 5.0 |
| Stannous Fluoride USP | 0.3 - 0.5 |
| Glycerin 96% USP | 5 - 20 |
| Sodium Carboxymethylcellulose | 0.5 - 5.0 |
| Flavor | 0.3 - 1.0 |
| Preservative | 0.1 - 1.0 |

For additional protection against the oxidation, the mouthwash product should be stored in containers which have low oxygen permeability and which preferably do not expose the contents to silica, silicates, oxygen, any other materials which would reduce the stability of the stannous ion, or introduce physiologically unacceptable substances into the contents. Preferred containers are bottles made of an oxygen impermeable organic polymer such as polyethylene phthalate.

A preferred mouthwash formulation can have the composition shown in Table A-2.

This invention is further described by the following specific but non-limiting examples. Percentages are weight percents unless otherwise indicated.

**TABLE A-2**

| Ingredients | Weight Percent |
|---|---|
| Purified Water USP | 80 - 98 |
| Stannous Chelating Copolymer | 0.1 - 5.0 |
| Stannous Fluoride | 0.05 - 0.5 |
| Tween-80^{a} | 0.1 - 2.0 |
| Pluoronic F127^{b} | 0.1 2.0 |
| Flavor | 0.5 - 2 |
| Glycerin | 1 - 10 |
| Sorbitol | 1 - 10 |
| Denatured Ethanol | 1 - 10 |
| Sodium Benzoate | 0.01 - .2 |
| PEG 40^{c} | 0.1 - 1.0 |
| Tween-80^{a} is polyoxyethylenesorbitan monooleate. Pluronic F127^{b} is a condensate of ethylene oxide with propylene oxide condensates of propylene glycol. PEG 40^{c} is sorbitan diisostearate available from Emery Industries. | |

Preferred toothpaste formulations have the compositions shown in Table A-3.

**TABLE A-3**

| Ingredients | Weight Percent |
|---|---|
| Purified Water USP | 10 - 30 |
| Carbopol^{a} | 0.05 - 1.0 |
| Sodium CMC^{b} | 0.1 - 2.0 |
| Glycerin 96% USP | 5.0 - 30 |
| Xanthan Gum (KELTROL) | 0.1 - 1.5 |
| Methylparaben NF | 0.1 - 5.0 |
| Propylparaben NF | 0.01 - .10 |
| Polyethylene Glycol 400^{c} | 0.1 - 4.0 |
| Stannous Chelating Copolymer | 0.5 - 5.0 |
| Stannous Fluoride | 0.3 - 0.5 |
| Sorbitol 70% USP | 10 - 35 |
| Sodium Saccharin USP Crystal | 0.05 - 0.5 |
| Calcium Pyrophosphate | 30 - 45 |
| Zinc Citrate | 0.1 - 2.0 |
| Insoluble Sodium Metaphosphate | 30 - 45 |
| Sodium Lauryl Sulfate Needles | 0.05 - 3.0 |
| Sodium Hydroxide NF (10% sol'n) | 0.5 - 5.0 |
| Flavor | 0.5 - 1.5 |
| Carbopol^{a} is a carboxyvinyl polymer available from B.F. Goodrich Company. Sodium CMC^{b} is carboxymethyl cellulose. Polyethylene Glycol 400^{c} is polyethylene glycol having a molecular weight of about 400, available from Union Carbide. | |

### EXAMPLE 1

An aqueous solution containing methyl vinyl ether-maleic anhydride copolymer and percent stannous fluoride is mixed with the other ingredients listed below to form a non-abrasive home treatment gel product having the following composition:

| Ingredients | Weight Percent |
|---|---|
| Purified Water USP | 18.00 |
| Sorbitol Solution (70%) USP | 66.85 |
| Copolymer^{a} | 1.00 |
| Stannous Fluoride USP | 0.44 |
| Glycerin 96% USP | 10.00 |
| Sodium Carboxymethylcellulose | 1.70 |
| Blue FD&C #1 (1% solution) | 0.90 |
| Yellow FD&C #10 (1% solution) | 0.61 |
| Flavor - Felton Spearmint HF Triple Distilled A-1895 | 0.50 |
| Copolymer^{a} is the free acid of a methyl vinyl ether and maleic anhydride copolymer having a molecular weight of about 30,000 to about 1,000,000. | |

### EXAMPLE 2

The procedure of Example 1 was repeated to form another non-abrasive home treatment gel including the following ingredients:

| Ingredients | Weight Percent |
|---|---|
| Purified Water USP | 18.00 |
| Sorbitol Solution (70%) USP | 66.235 |
| Copolymer^{a} | 1.00 |
| Stannous Fluoride USP | 0.44 |
| Glycerin 96 USP | 12.00 |
| Sodium Carboxymethylcellulose | 1.70 |
| Grape Flavor #11540 | 0.50 |
| Tartaric Acid | 0.125 |
| Copolymer^{a} is the free acid of a methyl vinyl ether and maleic anhydride copolymer having a molecular weight of about 30,000 to about 1,000,000. | |

### EXAMPLE 3

The non-abrasive home treatment gel of Example 1 was analyzed to determine the initial concentrations of stannous and fluoride ions achieved at formulation and to determine the initial ion concentrations after 1, 2, and 3 months storage at 45°C. in sealed oxygen impermeable tubes.

The following results were obtained, concentrations being reported as weight percents.

| Time | Sn as SnF₂ | F as Snf₂ | pH |
|---|---|---|---|
| Initial | 0.463 | 0.432 | 3.6 |
| one month | 0.412 | 0.421 | 3.8 |
| two months | 0.410 | 0.417 | 3.8 |
| three months | 0.398 | 0.405 | 3.8 |

### EXAMPLE 4

The non-abrasive home treatment gel of Example 2 was analyzed to determine the initial concentrations of stannous and fluoride ions achieved at formulation and to determine the initial ion concentrations after 1, 2, and 3 months storage at 45°C. in sealed oxygen impermeable tubes.

The following results were obtained, concentrations being reported as weight percents.

| Time | Sn as SnF₂ | F as Snf₂ | pH |
|---|---|---|---|
| Initial | 0.461 | 0.433 | 3.5 |
| one month | 0.423 | 0.418 | 3.6 |
| two months | 0.423 | 0.413 | 3.6 |
| three months | 0.413 | 0.409 | 3.7 |

### EXAMPLE 5

The following Example illustrates the stabilizing effect of the alkyl vinyl ether/maleic anhydride copolymers on stannous fluoride in an aqueous environment such as encountered during formulation of stannous fluoride containing oral care products. Two sample solutions of 0.455 percent by weight stannous fluoride in deionized water were prepared. In the first sample solution, the stannous fluoride was added to the deionized water. In a second sample solution, the stannous fluoride was added to a solution of 1 percent by weight of methyl vinyl ether-maleic anhydride copolymer in deionized water. In the first sample solution a precipitate of stannous oxides appeared almost immediately and the intensity of the precipitate increased with time. Analyses of the supernatant and sediment portions of centrifuged samples revealed that the concentration of stannous fluoride in the supernatant and sediment portions were 0.371 percent by weight and 0.099 percent by weight respectively. The second sample solution was clear and there was no evidence of precipitation. These results were confirmed by comparative sample analyses using a turbidity meter. Analyses of the supernatant and sediment portions of centrifuged samples revealed that the concentration of stannous fluoride in the supernatant was 0.456 percent by weight and no stannous fluoride was detected in the sediment portion.

### EXAMPLE 6

An aqueous solution containing methyl vinyl ether-maleic anhydride copolymer and percent stannous fluoride is prepared to form a stabilized stannous fluoride solution. This solution is mixed with the other ingredients listed in Table A-4. The amounts of the ingredients are selected to yield a final mouthwash composition having the following composition:

**TABLE A-4**

| Ingredients | Weight Percent |
|---|---|
| Purified Water | 70.75 |
| Glycerin 96% USP | 15.00 |
| Stannous Fluoride | 0.10 |
| Copolymer^{a} | 10.00 |
| Sodium phosphate, dibasic USP | 0.05 |
| FD2C Blue #1 (1% solution) | 0.05 |
| FD2C Yellow #10 (1% solution) | 0.15 |
| Ethyl Alcohol 95% USP | 2.50 |
| Methyl Paraben | 0.10 |
| Propyl Paraben | 0.01 |
| PEG 40^{b} | 0.30 |
| Spice Mint flavor (Noville # 30712) | 0.06 |
| Copolymer^{a} is the free acid of a methyl vinyl ether and maleic anhydride copolymer having a molecular weight of about 70,000, benzene-free, and sold under the tradename GANTREZ S-97BF by GAF Corporation. It is purchased in a liquid form containing 10 wt.% copolymer. PEG 40^{b} is sorbitan diisostearate available from Emery Industries. | |

### EXAMPLE 7

An aqueous solution containing methyl vinyl ether-maleic anhydride copolymer and percent stannous fluoride is prepared to form a stabilized stannous fluoride solution. This solution is mixed with the other ingredients listed in Table B below. The amounts of the ingredients are selected to yield a final toothpaste composition having the following composition:

**TABLE B**

| Ingredients | Weight Percent |
|---|---|
| Purified Water | 29.00 |
| Polyethylene Glycol 400 | 0.40 |
| Glycerin 96% USP | 5.00 |
| Methylparaben NF | 0.15 |
| Propylparaben NF | 0.05 |
| Copolymer^{a} | 1.00 |
| Stannous Fluoride | 0.455 |
| Sodium Hydroxide (10% solution) | 4.50 |
| Sorbitol 70% USP | 16.275 |
| Sodium Saccharin USP Crystal | 0.20 |
| Calcium Pyrophosphate | 40.00 |
| Zinc Citrate | 0.40 |
| Glycerine 96% USP | 5.00 |
| Natural Mint Flavor A-1159 FELTON | 1.00 |
| Sodium Lauryl Sulfate Needles | 1.00 |
| Copolymer^{a} is the free acid of a methyl vinyl ether and maleic anhydride copolymer having a molecular weight of about 70,000, benzene-free, and sold under the tradename GANTREZ S-97BF by GAF Corporation. | |

### EXAMPLE 8

Dentifrice compositions prepared as described in Example 7 and having the formulations shown in Table C were analyzed to determine the initial concentrations of stannous and fluoride ions achieved at formulation and to determine the initial ion concentrations after 1, 2, and 3 months storage at 45°C. in sealed oxygen impermeable tubes.

**TABLE C**

| Ingredients | Weight Percent | | | |
|---|---|---|---|---|
| | A | B | C | D |
| Purified Water | 29.00 | 29.00 | 29.00 | 29.00 |
| Carbopol 950 | 0.20 | 0.20 | 0.20 | 0.20 |
| Glycerin 96% USP | 10.00 | 10.00 | 10.00 | 10.00 |
| Xanthan Gum | 0.15 | 0.40 | 0.40 | 0.40 |
| Methylparaben NF | 0.15 | 0.15 | 0.15 | 0.15 |
| Propylparaben NF | 0.02 | 0.02 | 0.02 | 0.02 |
| Copolymer^{a} | 1.00 | 1.00 | 1.00 | 1.00 |
| Stannous Fluoride | 0.455 | 0.455 | 0.455 | 0.455 |
| Sodium Hydroxide (10% solution) | 4.50 | 4.50 | 4.50 | 4.50 |
| Sorbitol 70% USP | 21.225 | 10.275 | 16.675 | 16.675 |
| Sodium Saccharin USP Crystal | 0.20 | 0.20 | 0.20 | 0.20 |
| Calcium Pyrophosphate | 30.00 | 35.00 | 35.00 | 35.00 |
| Zinc Citrate | 0.80 | 0.80 | 0.40 | 0.40 |
| Natural Mint Flavor A-1159 FELTON | 1.00 | 1.00 | | |
| Doublemint Flavor #30672 Noville | | | 1.00 | 1.00 |
| Sodium Lauryl Sulfate Needles | 1.00 | 1.00 | 1.00 | 1.00 |
| Copolymer^{a} is the free acid of a methyl vinyl ether and maleic anhydride copolymer having a molecular weight of about 70,000, benzene-free, and sold under the tradename GANTREZ S-97BF by GAF Corporation. | | | | |

The following results were obtained, concentrations being reported as weight percents.

| Formulation A | | | |
|---|---|---|---|
| Time | Sn as SnF₂ | F as SnF₂ | pH |
| Initial | 0.453 | 0.420 | 5.2 |
| one month | 0.435 | 0.439 | 5.2 |
| two months | 0.458 | 0.428 | 5.1 |
| three months | 0.412 | 0.414 | 5.2 |

| Formulation B | | | |
|---|---|---|---|
| Time | Sn as SnF₂ | F as SnF₂ | pH |
| Initial | 0.479 | 0.430 | 5.1 |
| one month | 0.444 | 0.442 | 5.1 |
| two months | 0.402 | 0.398 | 5.2 |
| three months | 0.433 | 0.353 | 5.2 |

| Formulation C | | | |
|---|---|---|---|
| Time | Sn as SnF₂ | F as SnF₂ | pH |
| Initial | 0.454 | 0.452 | 5.3 |
| one month | 0.439 | 0.472 | 5.2 |
| two months | 0.468 | 0.472 | 5.2 |
| three months | 0.477 | 0.469 | 5.3 |

| Formulation D | | | |
|---|---|---|---|
| Time | Sn as SnF₂ | F as SnF₂ | pH |
| Initial | 0.447 | 0.429 | 5.7 |
| one month | 0.443 | 0.440 | 5.6 |
| two months | 0.455 | 0.420 | 5.8 |
| three months | 0.458 | 0.441 | 5.8 |

### EXAMPLE 9

Dentifrice compositions prepared as described in Example 7 and having the formulations shown in Table D were analyzed to determine the initial concentrations of stannous and fluoride ions achieved at formulation and to determine the initial ion concentrations after 1, 2, and 3 months storage at 45°C. in sealed oxygen impermeable tubes.

**TABLE D**

| Ingredients | Weight Percent | | | | |
|---|---|---|---|---|---|
| | E | F | G | H | I |
| Purified Water | 29.50 | 22.50 | 29.50 | 24.50 | 17.00 |
| Carbopol 950 | 0.30 | | 0.20 | 0.20 | |
| Glycerin 96% USP | 10.00 | 10.00 | 10.00 | 10.00 | 10.00 |
| Xanthan Gum | 0.50 | 0.60 | 0.80 | 0.80 | 0.70 |
| Carboxymethyl Cellulose | | 0.40 | | | 0.30 |
| Methylparaben NF | 0.15 | 0.15 | 0.15 | 0.15 | 0.15 |
| Propylparaben NF | 0.02 | 0.02 | 0.02 | 0.02 | 0.02 |
| Copolymer^{a} | 1.00 | 1.00 | 1.00 | 1.00 | 1.00 |
| Stannous Fluoride | 0.455 | 0.455 | 0.455 | 0.455 | 0.43 |
| Sodium Hydroxide (10% solution) | 4.50 | 3.00 | 4.50 | 4.00 | 3.00 |
| Sorbitol 70% USP | 14.975 | 24.275 | 14.975 | 15.875 | 24.40 |
| Sodium Saccharin USP Crystal | 0.20 | 0.20 | 0.20 | 0.20 | 0.20 |
| Calcium Pyrophosphate | 35.00 | 35.00 | 35.00 | 40.00 | 40.00 |
| Zinc Citrate | 0.40 | 0.40 | 0.40 | 0.80 | 0.80 |
| Tween-80^{b} | 1.0 | | | | |
| Pluronic F127^{c} | | | 0.80 | | |
| Mint Flavor A-1159 FELTON | 0.8 | 0.8 | 0.80 | 1.00 | 1.00 |
| Sodium Lauryl Sulfate Needles | 1.2 | 1.2 | 1.2 | 1.00 | 1.00 |
| Copolymer^{a} is the free acid of a methyl vinyl ether and maleic anhydride copolymer having a molecular weight of about 70,000, benzene-free, and sold under the tradename GANTREZ S-97BF by GAF Corporation. Tween-80^{b} is polyoxyethylenesorbitan monooleate. Pluronic F127^{c} is a condensate of ethylene oxide with propylene oxide condensates of propylene glycol. | | | | | |

The following results were obtained, concentrations being reported as weight percents.

| Formulation E | | | |
|---|---|---|---|
| Time | Sn as SnF₂ | F as SnF₂ | pH |
| Initial | 0.462 | 0.453 | 5.6 |
| one month | 0.432 | 0.449 | 5.6 |
| two months | 0.453 | 0.444 | 5.4 |
| three months | 0.431 | 0.453 | 5.4 |

| Formulation F | | | |
|---|---|---|---|
| Time | Sn as SnF₂ | F as SnF₂ | pH |
| Initial | 0.471 | 0.435 | 5.2 |
| one month | 0.390 | 0.441 | 5.2 |
| two months | 0.415 | 0.436 | 5.0 |
| three months | 0.418 | 0.429 | 5.0 |

| Formulation G | | | |
|---|---|---|---|
| Time | Sn as SnF₂ | F as SnF₂ | pH |
| Initial | 0.456 | 0.449 | 5.8 |
| one month | 0.445 | 0.439 | 5.7 |
| two months | 0.451 | 0.449 | 5.5 |
| three months | 0.454 | 0.430 | 5.4 |

| Formulation H | | | |
|---|---|---|---|
| Time | Sn as SnF₂ | F as SnF₂ | pH |
| Initial | 0.462 | 0.460 | 5.4 |
| one month | 0.464 | 0.438 | 5.1 |
| two months | 0.459 | 0.440 | 5.1 |
| three months | 0.450 | 0.445 | 4.9 |

| Formulation I | | | |
|---|---|---|---|
| Time | Sn as SnF₂ | F as SnF₂ | pH |
| Initial | 0.469 | 0.438 | 5.2 |
| one month | 0.396 | 0.407 | 4.9 |
| two months | 0.414 | 0.410 | 4.9 |
| three months | 0.398 | 0.409 | 4.6 |

## Claims

1. An aqueous stannous fluoride composition for oral care, containing stannous fluoride and alkyl (from 1 to 6 carbon atoms) vinyl ether and maleic anhydride or maleic acid copolymer in an amount sufficient to efficiently stabilize the stannous fluoride concentration, the composition being substantially free from soluble pyrophosphates, silica containing compounds and aldehyde containing flavoring agents.

2. A composition of claim 1, wherein the lower alkyl vinyl ether is methyl vinyl ether.

3. A composition of claim 1 or 2, wherein the composition is an aqueous non-abrasive home treatment gel composition.

4. A composition of claim 3, wherein the amount of stannous fluoride included in the composition provides from 0.05 to 5.0 percent by weight stannous fluoride, the copolymer being present in an amount from 0.5 to 5.0 percent by weight, the weight ratio of stannous fluoride to copolymer being from 0.01 to 1.0.

5. A composition of claim 1 or 2, wherein the composition is an aqueous mouthwash composition.

6. A composition of claim 5, wherein the amount of stannous fluoride included in the composition provides from 0.05 to 5.0 percent by weight stannous fluoride, the copolymer being present in an amount from 0.5 to 5.0 percent by weight, the weight ratio of stannous fluoride to copolymer being from 0.01 to 1.0.

7. A composition of claim 1 or 2, wherein the composition is an aqueous toothpaste composition.

8. A composition of claim 7, wherein the amount of stannous fluoride included in the composition provides from 0.05 to 5.0 percent by weight stannous fluoride, the copolymer being present in an amount from 0.5 to 5.0 percent by weight, the weight ratio of stannous fluoride to copolymer being from 0.05 to 1.0.

9. A composition of claim 7 or 8, wherein the composition includes an abrasive.

10. A composition of claim 9, wherein the abrasive is a calcium pyrophosphate.

11. A method for the preparation of an aqueous stannous fluoride composition for oral care, which comprises dispersing the stannous fluoride in an aqueous solution of an alkyl vinyl ether and maleic anhydride or maleic acid copolymer, the amount of said copolymer being sufficient to stabilize the stannous fluoride concentration during formulation.

12. A method of claim 11, wherein the alkyl vinyl ether is methyl vinyl ether.

13. A method of claim 11 or 12, wherein the amount of stannous fluoride included in the solution provides from 0.05 to 5.0 percent by weight stannous fluoride in the formulated composition.

## Patentansprüche

1. Zinn(II)fluorid enthaltende wäßrige Zusammensetzung für die Mundpflege, die außer Zinn(II)fluorid ein Copolymer aus einem Alkylvinylether (Alkyl mit 1 bis 6 Kohlenstoffatomen) und der Maelinsäure oder ihres Anhydrids in einer Menge enthält, die zum wirksamen Stabilisieren der Konzentration des Zinn(II)fluorids genügt, wobei die Zusammensetzung im wesentlichen frei ist von löslichen Pyrophosphaten, siliciumdioxidhaltigen Verbindungen und aldehydhaltigen Geschmacksstoffen.

2. Zusammensetzung nach Anspruch 1, in der der niedere Alkylvinylether Methvlvinylether ist.

3. Zusammensetzung nach Anspruch 1 oder 2, die eine nicht schleifend oder scheuernd wirkende, wäßrige Zusammensetzung für die häusliche Verwendung sit.

4. Zusammensetzung anch Anspruch 3, die 0,05 bis 5,0 Gew.-% Zinn(II)fluorid enthält und in der das Copolymer in einer Menge von 0,5 bis 5,0 Gew.-% enthalten ist, wobei das Gewichtsverhältnis von Zinn(II)fluorid zu Copolymer zwischen 0,01 und 1,0 beträgt.

5. Zusammensetzung nach Anspruch 1 oder 2, die eine wäßrige Zusammensetzung zum Mundspülen ist.

6. Zusammensetzung nach Anspruch 5, die 0,05 bis 5,0 Gew.-% Zinn(II)fluorid enthält und in der das Copolymer in einer Menge von 0,5 bis 5,0 Gew.-% enthalten ist, wobei das Gewichtsverhältnis von Zinn(II)fluorid zu Copolymer zwischen 0,01 und 1,0 beträgt.

7. Zusammensetzung nach Anspruch 1 oder 2, die eine wäßrige Zahnpasta ist.

8. Zusammensetzung nach Anspruch 7, die 0,05 bis 5,0 Gew.-% Zinn(II)fluorid enthält und in der das Copolymer in einer Menge von 0,5 bis 5,0 Gew.-% enthalten ist, wobei das Gewichtsverhältnis von Zinn(II)fluorid zu Copolymer zwischen 0,01 und 1,0 beträgt.

9. Zusammensetzung nach Anspruch 7 oder 8, die ein Schleif- oder Scheuermittel enthält.

10. Zusammensetzung nach Anspruch 9, in der das Schleif- oder Scheuermittel Calciumpyrophosphat ist.

11. Verfahren zum Herstellen einer Zinn(II)fluorid enthaltenden wäßrigen Zusammensetzung für die Mundpflege, in dem das Zinn(II)fluorid in einer wäßrigen Lösung eines Copolymers aus einem Alkylvinylether und der Maleinsäure oder ihrem Anhydrid dispergiert wird, wobei die Menge des Copolymers zum Stabilisieren der Konzentration des Zinn(II)fluorids während der Herstellung genügt.

12. Verfahren nach Anspruch 11, in dem der Alkylvinylether Methylvinylether ist.

13. Verfahren nach Anspruch 11 oder 12, in dem die Lösung Zinn(II)fluorid in einer solchen Menge enthält, daß die angesetzte Zusammensetzung 0,05 bis 5,0 Gew.-% Zinn(II)fluorid enthält.

## Revendications

1. Composition aqueuse de fluorure stanneux destinée à des soins buccaux, contenant du fluorure stanneux et de l'éther vinylique d'alkyle en C₁-C₆ et un copolymère d'anhydride maléique ou d'acide maléique en une quantité suffisante pour stabiliser efficacement la concentration de fluorure stanneux, la composition étant pratiquement exempte de pyrophosphates solubles, de composés contenant de la silice et d'arômes contenant un aldéhyde.

2. Composition selon la revendication 1, dans laquelle l'éther vinylique d'alkyle inférieur est l'éther vinylique de méthyle.

3. Composition selon la revendication 1 ou la revendication 2, dans laquelle la composition est une composition aqueuse de gel non abrasif pour traitement à domicile.

4. Composition selon la revendication 3, dans laquelle la quantité de fluorure stanneux comprise dans la composition assure entre 0,05 et 5,0 % en masse de fluorure stanneux, le copolymère étant présent à raison de 0,5 à 5,0 % en masse, le rapport massique du fluorure stanneux au copolymère étant compris entre 0,01 et 1,0.

5. Composition selon la revendication 1 ou la revendication 2, dans laquelle la composition est une composition aqueuse de type bain de bouche.

6. Composition selon la revendication 5, dans laquelle la quantité de fluorure stanneux comprise dans la composition assure entre 0,05 et 5,0 % en masse de fluorure stanneux, le copolymère étant présent à raison de 0,5 à 5,0 % en masse, le rapport massique du fluorure stanneux au copolymère étant compris entre 0,01 et 1,0.

7. Composition selon la revendication 1 ou la revendication 2, dans laquelle la composition est une composition aqueuse de type dentifrice.

8. Composition selon la revendication 7, dans laquelle la quantité de fluorure stanneux comprise dans la composition assure entre 0,05 et 5,0 % en masse de fluorure stanneux, le copolymère étant présent à raison de 0,5 à 5,0 % en masse, le rapport massique du fluorure stanneux au copolymère étant compris entre 0,05 et 1,0.

9. Composition selon la revendication 7 ou la revendication 8, dans laquelle la composition comprend un abrasif.

10. Composition selon la revendication 9, dans laquelle l'abrasif est un pyrophosphate de calcium.

11. Procédé de préparation d'une composition aqueuse de fluorure stanneux destinée à des soins buccaux, qui comprend la dispersion du fluorure stanneux dans une solution aqueuse d'un éther vinylique d'alkyle et d'un copolymère d'anhydride maléique ou d'acide maléique, la quantité dudit copolymère étant suffisante pour stabiliser la concentration de fluorure stanneux lors de la formulation.

12. Procédé selon la revendication 11, dans lequel l'éther vinylique d'alkyle est l'éther vinylique de méthyle.

13. Procédé selon la revendication 11 ou la revendication 12, dans lequel la quantité de fluorure stanneux comprise dans la solution assure entre 0,05 et 5,0 % en masse de fluorure stanneux dans la composition formulée.
